# EUROPÄISCHE PATENTANMELDUNG

(11) **EP 1 806 527 A2**
(43) Veröffentlichungstag der Anmeldung: **11.07.2007**
(21) Anmeldenummer: 06025333.3
(22) Anmeldetag: 07.12.2006
(51) Int. Cl.: F16L 33/22

(54) **Schlauchanschluss für niedrige Drücke**

(30) Priorität: 09.01.2006 DE 102006001326
(71) Anmelder: Tecno Plast Industrietechnik GmbH, 40549 Düsseldorf (DE)
(72) Erfinder: Mayer, Lorenz, 45476 Mülheim (DE); Schnitzler, Albert, 52379 Langerwehe (DE)
(74) Vertreter: Gerber, Wolfram

(57) **Zusammenfassung**

Die Erfindung betrifft einen Schlauchanschluss, ein Verbindungsstück (1) aufweisend, welches einen in dem freien Ende des Schlauches einliegenden Stutzen (1b) hat, dessen außerhalb des Schlauches liegender Teil als Flansch (1a) ausgebildet ist und einer das freie Ende des Schlauches (3a) umgreifenden rohrförmigen Hülse (2), wobei das freie Ende (3a) des Schlauches (3) zwischen dem Stutzen (1b) und der Hülse (2) klemmend einliegt, wobei zumindest der Stutzen (1b) und der Bereich (2a) der Hülse (2), welcher bei montiertem Schlauchanschluss den Stutzen (1b) umgreift, ihre Form bei der Montage nicht verändern.

## Beschreibung

Die vorliegende Erfindung betrifft einen Schlauchanschluss nach dem Oberbegriff des Anspruch 1.

Schlauchanschlüsse sind hinlänglich bekannt. so wird meist ein Verbindungsstück, welches in der Regel mit seiner dem Schlauch abgewandten Seite ein Kupplungsstück zum Anschluss eines weiteren Kupplungsteils bildet, in das Schlauchende mit einem Stutzen eingeschoben. Der Stutzen weist an seiner äußeren Mantelfläche meist umlaufende sägezahnartige Vorsprünge auf, die ein ungewolltes Herunterrutschen des Schlauchendes vom Stutzen verhindern. Ein derartiger Schlauchanschluss ist z.B. aus der JP 2005106175 bekannt. Optional kann noch eine Überwurfmutter den Schlauch zwischen Stutzen und Überwurfmutter einklemmen, wie es z.B. aus der EP 0 829 670 bekannt ist.

Der Schlauchanschluss gem. der JP 200506175 weist den Nachteil auf, dass nur ein spezieller Schlauch sicher mit dem Verbindungsteil zusammenwirkt. Der Schlauchanschluss ist nicht geeignet, für z.B. Siliconschläuche. Der Schlauchanschluss gem. der EP 0 829670 weist den Nachteil auf, dass die Überwurf- und Klemmmutter aufwendig gestaltet ist.

Aufgabe der vorliegenden Erfindung ist es, insbesondere für weiche Siliconschläuche einen lösbaren Schlauchanschluss bereitzustellen, welcher für niedrige Drücke geeignet ist, aus einfachen Teilen besteht und eine möglichst glatte Oberflächenkontur aufweist, so dass er einfach zu reinigen und desinfizieren ist.

Diese Aufgabe wird vorteilhaft mit einem Schlauchanschluss mit den Merkmalen des Anspruch 1 gelöst. Weitere vorteilhafte Ausgestaltungen ergeben sich durch die Merkmale der Unteransprüche. Ebenso wird eine Vorrichtung zur Montage des erfindungsgemäßen Schlauchanschlusses beansprucht, mittels derer der Schlauchanschluss einfach und zuverlässig insbesondere in Verbindung mit einem Siliconschlauch montierbar ist.

Der erfindungsgemäße Schlauchanschluss zeichnet sich vorteilhaft dadurch aus, dass der Schlauch lediglich durch eine Klemmung mittels der Hülse auf dem Verbindungsstück gehalten ist. Die Klemmverbindung kann ohne Werkzeuge gelöst werden, in dem am Verbindungsstück und dem Schlauch gezogen wird. Hierdurch wird der Schlauch und auch das zwischen dem Verbindungsstück und der Hülse eingeklemmte Schlauchende gelängt, wodurch sich die Wandstärke des Schlauchendes verringert. Die Klemmung wird hierdurch gelockert, so dass das Schlauchende nebst der Hülse von dem Verbindungsstück heruntergezogen werden kann. Der erfindungsgemäße Schlauchanschluss kann für drucklosen Betrieb oder Niederdruckbetrieb von einigen bar, insbesondere bis zu 3 bar, sicher verwendet werden.

Sofern der Schlauchanschluss mit der erfindungsgemäßen Vorrichtung montiert wird, werden die einzelnen Teile nicht beschädigt, so dass sie vorteilhaft mehrfach verwendet werden können.

Das Verbindungsstück sowie die Hülse sind einfach zu fertigende Teile, wobei keine aufwendigen Gewinde in das Verbindungsstück und/oder die Hülse geschnitten werden müssen. Durch geeignete Wahl der Durchmesserverhältnisse bei vorgegebener Wandstärke des flexiblen Schlauchs ist es möglich, die Klemmkraft genau vorzugeben, so dass der Schlauch bei Belastung mit dem Betriebsdruck noch sicher im Schlauchanschluss geklemmt ist.

Nachfolgend wird anhand von Zeichnungen eine mögliche Ausführungsform des erfindungsgemäßen Schlauchanschlusses sowie eine Vorrichtung zur Montage sowie Demontage des Schlauchanschlusses näher erläutert.

Es zeigen:
- Fig. 1:: Explosionszeichnung eines erfindungsgemäßen Schlauchanschlusses mit zugehörigem Schlauchende;
- Fig. 2:: Vorrichtung zur Montage eines erfindungsgemäßen Schlauchanschlusses;
- Fig. 3:: per Hand vormontierter Schlauchanschluss, eingesetzt in die Vorrichtung gemäß Figur 2;

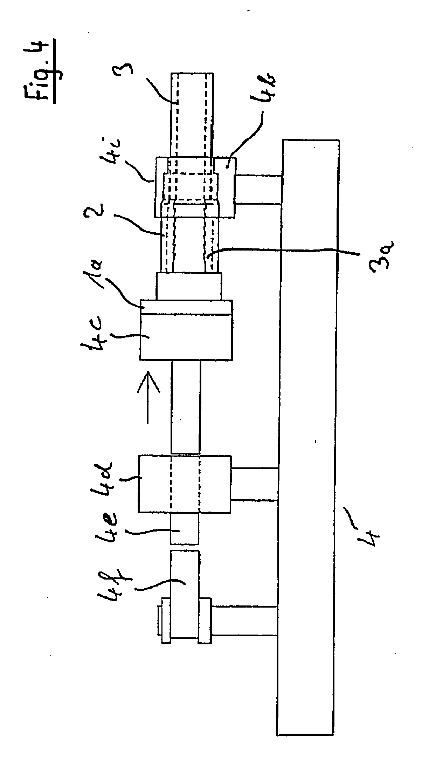

- Fig. 4:: Montage des erfindungsgemäßen Schlauchanschlusses gemäß Figur 1 mit der Vorrichtung gem. Figur 2;
- Fig. 5:: fertig montierter erfindungsgemäßer Schlauchanschluss;
- Fig. 6:: Demontage eines montierten erfindungsgemäßen Schlauchanschlusses.

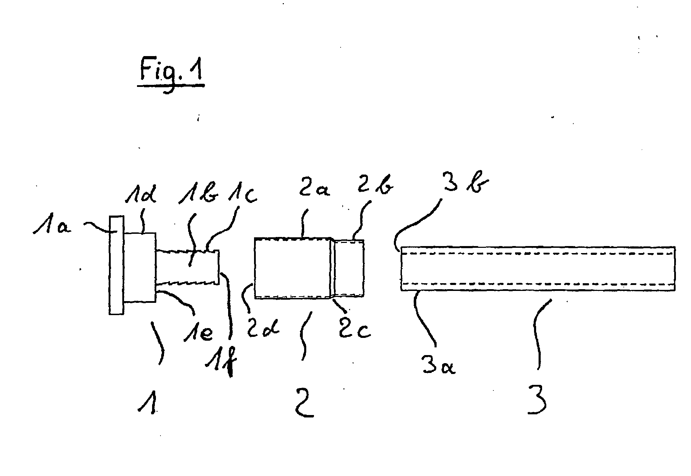

Die Figur 1 zeigt eine Explosionszeichnung des erfindungsgemäßen Schlauchanschlusses, welcher aus einem Verbindungsstück 1, einer Hülse 2 sowie dem Schlauch 3 besteht. Das Verbindungsstück 1 weist einen Flansch 1a auf, an den ein zylindrischer Bereich 1d angeformt ist, an dem wiederum ein Stutzen 1b angrenzt, welcher eine sägezahnförmige Ausgestaltung an seiner Außenmantelfläche aufweist. Der Außendurchmesser der Sägezahnverzahnung 1c ist derart bemessen, dass das Schlauchende 3a nur mit Kraft über den Stutzen 1b per Hand aufschiebbar ist. Die Hülse 2 weist zwei zylindrische Abschnitte 2a und 2b auf, wobei der Bereich 2a einen größeren Außen- und Innendurchmesser aufweist als der Bereich 2b. Beide Bereiche 2a und 2b sind über eine schräge Wandung 2c miteinander in Verbindung. Der Innendurchmesser des Bereichs 2b ist derart bemessen, dass er bei eingeschobenem Schlauch 3 eng an der Außenwandung des Schlauches 3 anliegt, diesen jedoch nicht zu stark deformiert, so dass der Innendurchmesser des Schlauches sich durch das Aufschieben der Hülse nicht oder nur unwesentlich verändert. Die Hülse weist eine Stirnseite 2d auf, welche bei fertig montiertem Schlauchanschluss zur Anlage an die Stirnwandung 1e des Abschnittes 1d des Verbindungselementes 1 gelangt.

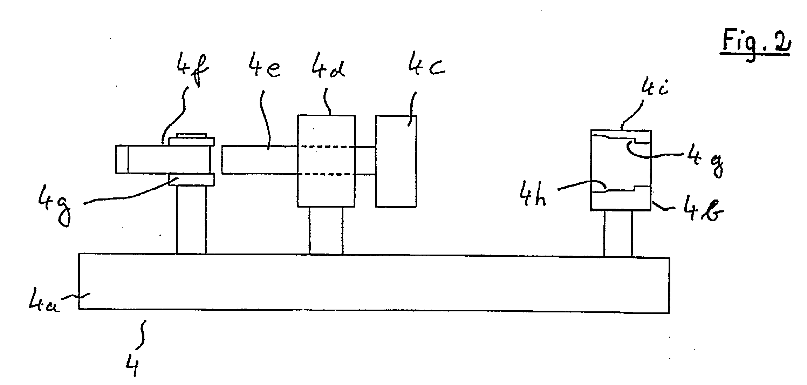

Die Figur 2 zeigt eine erfindungsgemäße Vorrichtung zur Montage des Schlauchanschlusses gemäß Figur 1, bestehend aus einer Basisplatte 4a, auf der ein Lager 4d für eine Kolbenstange 4e angeordnet ist. Die Kolbenstange 4e ist mit einem Kolben 4c in Verbindung, welcher über einen Antrieb 4f in Richtung des Gegenlagers 4b verstellbar ist. Der Antrieb ist drehbar bei 4g gelagert. Das Gegenlager 4b weist Anlageflächen 4g und 4h für die Hülse 2 des Schlauchanschlusses auf. Das Gegenlager 4b ist an seiner Oberseite geschlitzt und weist eine Längsöffnung 4i auf, durch die der Schlauch von oben in das Gegenlager 4b einführbar ist.

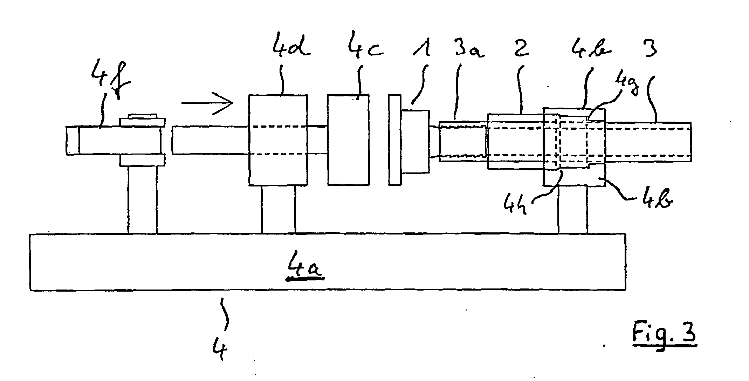

Die Figur 3 zeigt die Vorrichtung 4 mit eingesetztem Verbindungsstück 1, auf das bereits das Schlauchende 3a aufgezogen ist, wobei vorher die Hülse 2 über das Schlauchende 3a geschoben worden ist. Die Hülse 2 liegt mit ihrem im Durchmesser kleineren Bereich 2b in dem Widerlager 4b ein und stützt sich an den Widerlagerflächen 4g und 4h ab, sobald der Kolben 4c in Pfeilrichtung mittels des Antriebs 4f, welcher z. B. als Hebel ausgebildet ist, gegen das Verbindungsstück 1 drückt. Durch Halten des Schlauches 3 kann erreicht werden, dass beim Andrücken des Kolbens 4c gegen das Verbindungsstück 1 sowohl die Hülse 2 als auch der Schlauch jeweils mit ihren Stirnseiten gleichzeitig an die Stirnwandung 1e des Bereiches 1d des Verbindungsstücks 1 anschlagen, wonach dann die Montage des Schlauchanschlusses beendet ist. Der Kolben 4c muss anschließend lediglich nach links verstellt werden, damit die Hülse 2 mit dem Schlauch 3 zuerst ein wenig nach links bewegt werden kann, so dass dann der Schlauch 3 durch die obere Längsöffnung 4i des Widerlagers 4b aus der Vorrichtung 4 herausgenommen werden kann.

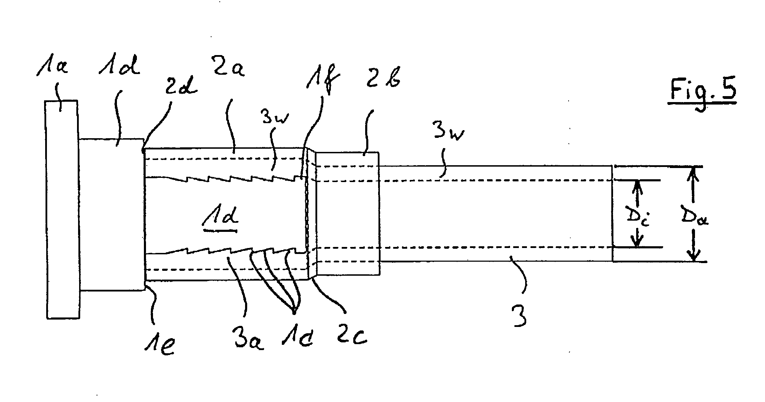

Die Figur 5 zeigt den fertig montierten Schlauchanschluss bestehend aus Schlauch 3, Hülse 2 sowie Verbindungsstück 1, wobei letzteres mittels seines Flansches 1a zum Anschluss an eine beliebige Vorrichtung oder an einen weiteren Verbindungsflansch 1a eines weiteren Schlauchanschlusses dient. Es ist selbstverständlich möglich, das Verbindungsstück anders auszugestalten, damit ein Ankuppeln an andere Geräte oder Anschlüsse möglich ist. Der Außendurchmesser des Stutzens 1b ist derart bemessen, dass bei aufgeschobenem Schlauchende 3a und entsprechend montierter Hülse 2 die Wandung 3w des Schlauches 3 zusammengedrückt ist, so dass aufgrund der Verzahnung das Schlauchende 3a sicher zwischen Stutzen 1b und Hülsenbereich 2a eingeklemmt ist. Der im Außen- und Innendurchmesser kleinere Bereich 2b der Hülse 2 stellt sicher, dass kein Schmutz zwischen die Hülse und den Schlauch 3 gelangen kann. Hierzu ist der Innendurchmesser des Hülsenbereiches 2b geringfügig kleiner als der Außendurchmesser DA des Schlauches 3. Es ist selbstverständlich möglich, die Rückhalteelemente 1c, welche als Sägezähne in Figur 5 ausgebildet sind, durch anders gestaltete Rücksprünge auszugestalten.

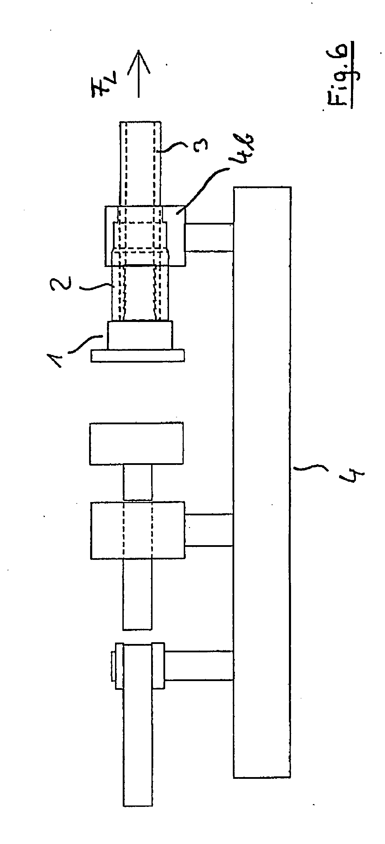

Figur 6 zeigt eine mögliche Art der Demontage des Schlauchanschlusses, wobei der Schlauchanschluss in das Widerlager 4b der Vorrichtung eingesetzt wird und anschließend am Schlauch 3 mit einer Kraft F_{L} in Pfeilrichtung gezogen wird. Hierdurch längt sich der Schlauch 3 und insbesondere das Schlauchende 3a, wodurch die Wandung 3w dünner wird und somit die Klemmung zwischen Stutzen 1d und Hülsenbereich 2a aufgehoben wird, so dass der Schlauch 3 vom Stutzen abgezogen werden kann. Nach der Demontage sind zumindest das Verbindungsstück 1 und die Hülse 2 wiederverwendbar.

Es ist selbstverständlich möglich, statt des manuellen Antriebes 4f bei der Vorrichtung einen elektromotorischen oder hydraulischen oder pneumatisch wirkenden Antrieb vorzusehen. Auch ist es möglich, statt einer fest installierten Vorrichtung eine mobile Vorrichtung zur Montage und Demontage des erfindungsgemäßen Schlauchanschlusses vorzusehen.

## Patentansprüche

1. Schlauchanschluss, ein Verbindungsstück (1) aufweisend, welches einen in dem freien Ende des Schlauches einliegenden Stutzen (1b) hat, dessen außerhalb des Schlauches liegender Teil als Flansch (1a) ausgebildet ist und einer das freie Ende des Schlauches (3a) umgreifenden rohrförmigen Hülse (2), **dadurch gekennzeichnet, dass** das freie Ende (3a) des Schlauches (3) zwischen dem Stutzen (1b) und der Hülse (2) klemmend einliegt, wobei zumindest der Stutzen (1b) und der Bereich (2a) der Hülse (2), welcher bei montiertem Schlauchanschluss den Stutzen (1b) umgreift, ihre Form bei der Montage nicht verändern.

2. Schlauchanschluss nach Anspruch 1, **dadurch gekennzeichnet, dass** die Wanddicke zumindest des freien Endes (3a) des unbelasteten bzw. nicht montierten Schlauches (3) größer ist als der radiale Spalt zwischen der äußeren Mantelfläche des Stutzens (1b) und der Innenwandung des Bereichs (2a) der Hülse (2), der den Stutzen umgreift.

3. Schlauchanschluss nach einem der vorhergehenden Ansprüche, **dadurch gekennzeichnet , dass** der Stutzen (1b) an seiner äußeren Mantelfläche mindestens einen Rückhaltevorsprung (1c) hat, der insbesondere durch einen umlaufenden Kragen gebildet ist.

4. Schlauchanschluss nach Anspruch 3, **dadurch gekennzeichnet, dass** Rückhaltevorsprung (1c) ein umlaufender zahnförmig ausgebildeter Kragen ist.

5. Schlauchanschluss nach Anspruch 3 oder 4, **dadurch gekennzeichnet , dass** zwischen dem mit insbesondere umlaufenden Rückhaltevorsprüngen ausgebildeten Bereich (1b) des Stutzens und dem Flansch (1a) ein Zwischenbereich (1d) angeordnet ist, der einen größeren Durchmesser als der mit den Rückhaltevorsprüngen versehene Bereich hat und dessen Durchmesser kleiner als der des Flansches ist.

6. Schlauchanschluss nach Anspruch 5, **dadurch gekennzeichnet, dass** der Zwischenbereich mit seiner dem mit Rückhaltevorsprüngen ausgebildeten Bereich zugewandten Stirnseite eine Anstoßfläche für die Stirnseite des freien Ende des Schlauches sowie der Hülse bildet.

7. Schlauchanschluss nach einem der vorhergehenden Ansprüche, **dadurch gekennzeichnet, dass** der Schlauch ein nicht armierter Siliconschlauch ist.

8. Schlauchanschluss nach einem der vorhergehenden Ansprüche, **dadurch gekennzeichnet , dass** die Hülse (2) einen ersten, den Stutzen (1b) umfassenden Bereich (2a) hat, der mindestens so lang ist, wie der mit Rückhaltevorsprüngen versehenen Bereich (1b) des Stutzens.

9. Schlauchanschluss nach Anspruch 8, **dadurch gekennzeichnet, dass** sich an den ersten Bereich (2a) der Hülse (2) ein Übergangsbereich (2c) mit sich hin zum zweiten Bereich (2b) verjüngendem Innendurchmesser anschließt.

10. Schlauchanschluss nach Anspruch 9, **dadurch ge**- **kennzeichnet**, dass der Innendurchmesser des zweiten Bereichs (2b) der Hülse (2) kleiner oder gleich dem Außendurchmesser (Dₐ) des unbelasteten Schlauchs (3) ist.

11. Verfahren zur Montage des Schlauchanschlusses nach einem der vorhergehenden Ansprüche, **dadurch gekennzeichnet, dass** in einem ersten Arbeitsschritt die Hülse (2) über das freie Schlauchende (3a) geschoben wird, wobei das freie Ende (3a) des Schlauches (3) mindestens mit einer Länge aus der Hülse (2) herausragt, welche der Länge des Stutzens (1b) entspricht, auf den das freie Ende (3a) im nachfolgenden zweiten Arbeitsschritt zumindest zum Teil aufgeschoben wird, wonach anschließend in einem dritten Arbeitsschritt die Hülse (2) über den Stutzen (1b) geschoben wird, bis die Hülse (2) sowie das freie Ende (3a) des Schlauches (3) vollständig den Bereich mit den Rückhaltevorsprüngen des Stutzens umgreift.

12. Verfahren nach Anspruch 11, **dadurch gekennzeichnet, dass** der dritte Arbeitsschritt mit einer Vorrichtung durchgeführt wird, bei der sich die Hülse (2) mit ihrem dem Flansch des Verbindungsstücks abgewandten Ende an einem Gegenlager abstützt und der Flansch mittels eines Aktuators in Richtung Hülse druckbeaufschlagt wird.

13. Verfahren nach Anspruch 12, **dadurch gekennzeichnet, dass** das während der Druckbeaufschlagung der Schlauch unter Zugspannung gehalten wird, derart, dass die Hülse und des freie Ende des Schlauches gleichzeitig in ihre Endposition gelangen.

14. Vorrichtung zur Durchführung des dritten Arbeitsschrittes des Verfahrens nach den Ansprüchen 11 bis 14, **dadurch gekennzeichnet , dass** die Vorrichtung ein Gegenlager für die Hülse aufweist, wobei das Gegenlager nach einer Seite eine Öffnung hat, derart, dass der Schlauch quer zu seiner Längserstreckung durch die Öffnung des Gegenlagers herausgeführt werden kann, wobei das Gegenlager eine insbesondere konische Anlagefläche für die Hülse aufweist, und dass die Vorrichtung ferner einen Kolben aufweist, der manuell, elektrisch, pneumatisch oder hydraulisch angetrieben ist.

15. Vorrichtung nach Anspruch 14, **dadurch gekennzeichnet, dass** der Kolben über eine Hebel manuell verstellbar ist.

16. verfahren zur Demontage des Schlauchanschlusses nach einem Ansprüche 1 bis 10, **dadurch gekennzeichnet, dass** der Schlauchanschluss an seinem Flansch gehalten und der Schlauch in die Länge gezogen wird, derart, dass sich die Wandstärke des zwischen der Hülse und dem Stutzen einliegenden Schlauchbereichs durch die Längung des Schlauches verringert und so das Schlauchende von dem Stutzen ziehbar ist.

17. Verfahren zur Demontage des Schlauchanschlusses nach Anspruch 16, **dadurch gekennzeichnet, dass** der Flansch von einer Haltevorrichtung in Position und der Schlauch mittels eines Greifmittels gehalten wird, wobei das Greifmittel mittels eines Antriebs vom flansch wegbewegbar ist, so dass das Schlauchende vom Stutzen gezogen wird.
